# FASCICULE DE BREVET EUROPEEN

(11) **EP 3 440 727 B1**
(45) Date de publication et mention de la délivrance du brevet: **24.06.2020**
(21) Numéro de dépôt: 17715680.9
(22) Date de dépôt: 03.04.2017
(51) Int. Cl.: H01M 4/90, H01M 4/92, B01J 23/42, B01J 37/00, B01J 37/02, B01J 37/16, B01J 37/34, C08F 12/30, B01J 37/06

(54) **PROCEDE DE PREPARATION DE PARTICULES DE PLATINE CONDUCTRICES DE PROTONS A FORTE SURFACE ACTIVE GREFFEES A LEUR SURFACE PAR DES POLYMERES CONDUCTEURS DE PROTONS SPECIFIQUES**
VERFAHREN ZUR HERSTELLUNG PROTONENLEITENDER PLATINPARTIKEL MIT EINEM GROSSEN AKTIVEN BEREICH UND MIT SPEZIFISCHEN PROTONENLEITENDEN POLYMEREN GEPFROPFTE OBERFLÄCHE
METHOD FOR PRODUCING PROTON-CONDUCTING PLATINUM PARTICLES WITH A LARGE ACTIVE SURFACE AREA AND SURFACE-GRAFTED WITH SPECIFIC, PROTON-CONDUCTING POLYMERS

(30) Priorité: 04.04.2016 FR 1652936
(43) Date de publication de la demande: 13.02.2019
(73) Titulaire: Commissariat à l'énergie atomique et aux énergies alternatives, 75015 Paris (FR)
(72) Inventeur: BUVAT, Pierrick, 37250 Montbazon (FR); BIGARRÉ, Janick, 37000 Tours (FR); COUTANCEAU, Christophe, 86000 Poitiers (FR); BARANTON, Stève, 86000 Poitiers (FR); DRU, Delphine, 37250 Sorigny (FR)
(74) Mandataire: Brevalex
(86) Numéro de dépôt international: PCT/EP2017/057879
(87) Numéro de publication internationale: WO 2017/174524

(56) Documents cités:
- FR-A1- 2 982 173
- FR-A1- 2 982 264
- ANNE-CLAIRE FERRANDEZ ET AL: "Chemical Functionalization of Carbon Supported Metal Nanoparticles by Ionic Conductive Polymer via the "Grafting From" Method", CHEMISTRY OF MATERIALS, vol. 25, no. 19, 8 octobre 2013 (2013-10-08), pages 3797-3807, XP055308857, US ISSN: 0897-4756, DOI: 10.1021/cm401264f
- ANNE-CLAIRE FERRANDEZ ET AL: "Pt Particles Functionalized on the Molecular Level as New Nanocomposite Materials for Electrocatalysis", LANGMUIR, vol. 28, no. 51, 21 décembre 2012 (2012-12-21), pages 17832-17840, XP055308876, US ISSN: 0743-7463, DOI: 10.1021/la303588t
- DELPHINE DRU ET AL: "Fluorine-Free Pt Nanocomposites for Three-Phase Interfaces in Fuel Cell Electrodes", ACS CATALYSIS, vol. 6, no. 10, 7 octobre 2016 (2016-10-07), pages 6993-7001, XP055308874, US ISSN: 2155-5435, DOI: 10.1021/acscatal.6b02145
- SRINIVASAN HARISH ET AL: "Microwave assisted polyol method for the preparation of Pt/C, Ru/C and PtRu/C nanoparticles and its application in electrooxidation of methanol", JOURNAL OF POWER SOURCES, ELSEVIER SA, CH, vol. 214, 1 avril 2012 (2012-04-01), pages 33-39, XP028501982, ISSN: 0378-7753, DOI: 10.1016/J.JPOWSOUR.2012.04.045 [extrait le 2012-04-25]

## Description

### DOMAINE TECHNIQUE

La présente invention se rapporte à un procédé de préparation de particules de platine à surface active élevée, ces particules étant, de plus, conductrices de protons grâce à une fonctionnalisation desdites particules avec des polymères organiques conducteurs de protons spécifiques.

Ces particules ont pour caractéristiques de présenter une activité catalytique (notamment, pour l'oxydation de l'hydrogène ou la réduction de l'oxygène) tout en présentant une conductivité protonique.

De ce fait, ces particules trouvent leur application dans l'élaboration de matériaux d'électrodes, en particulier de matériaux destinés à entrer dans la constitution de couches catalytiques d'électrodes pour piles à combustible, telles que les piles fonctionnant à H₂/air ou à H₂/O₂ (connues sous l'abréviation PEMFC signifiant « Proton Exchange Membrane Fuel Cell »).

Ainsi, la présente invention se situe dans le domaine des piles à combustible fonctionnant sur le principe de l'oxydation de l'hydrogène et la réduction de l'oxygène.

### ETAT DE LA TECHNIQUE ANTÉRIEURE

Une pile à combustible est un générateur électrochimique, qui convertit l'énergie chimique en énergie électrique grâce à deux réactions électrochimiques : une réaction d'oxydation à l'anode d'un combustible (l'hydrogène) combinée à une réaction de réduction à la cathode d'un comburant (l'air ou l'oxygène).

Classiquement, ce type de piles à combustible comporte une pluralité de cellules électrochimiques montées en série, chaque cellule comprenant deux électrodes de polarité opposée séparées par une membrane échangeuse de protons faisant office d'électrolyte solide, cette membrane assurant le passage vers la cathode des protons formés, par réaction électrochimique, lors de l'oxydation du combustible à l'anode.

Les réactions électrochimiques susmentionnées (oxydation et réduction) se produisent au niveau de zones spécifiques des électrodes (dites, également, zones actives correspondant structurellement à des couches catalytiques) qui forment la jonction entre la couche de diffusion (au niveau de laquelle se produit l'alimentation en réactifs) des électrodes et la membrane et nécessitent, pour se produire, l'utilisation de catalyseurs, qui consistent, classiquement, pour les piles du type PEMFC, en des particules de platine.

Compte tenu des coûts impliqués par la présence d'un catalyseur comme le platine, il convient d'obtenir un maximum de surface catalytique pour une masse donnée de métal, un tel objectif pouvant être atteint par des particules de platine de tailles nanométriques (dénommées également nanoparticules de platine).

Il convient également, pour que les réactions électrochimiques puissent avoir lieu, que les particules de platine soient en contact à la fois avec du combustible ou du comburant (selon que l'on se situe au niveau de l'anode ou de la cathode), du conducteur protonique constitutif de la membrane et du conducteur électronique entrant dans la constitution de l'électrode (ce conducteur électronique étant classiquement un matériau carboné), cette zone de contact étant connue sous l'appellation de point triple, l'électrode étant d'autant plus efficace que le nombre de points triples est élevé.

En d'autres termes, en ces points triples, se présentent au niveau des particules de platine :
- une continuité physique avec la membrane électrolytique, pour assurer une conduction des protons H⁺;
- une continuité physique avec le conducteur électronique, pour assurer la conduction des électrons ; et
- une continuité physique avec la zone de diffusion des électrodes, pour assurer la diffusion des gaz (l'oxygène ou l'hydrogène pour les piles PEMFC).

Le maintien dans le temps de ces points triples suppose le respect de l'intégrité des zones de contacts entre les différents éléments entrant dans la constitution de ces points triples, ce qui implique un maintien de l'intégrité physique de ces différents éléments, notamment des particules de platine.

Or certaines études ont montré qu'il est possible d'assister, en cours de fonctionnement d'une pile, à une dégradation des particules de platine (induisant, de ce fait, une diminution de surface active) soit par des phénomènes de dissolution ou des phénomènes d'augmentation de tailles de particules (découlant, classiquement, de phénomènes d'agglomération).

Ces phénomènes de dissolution peuvent se produire avec des piles fonctionnant à très faibles pH (par exemple, un pH inférieur à 1) et à potentiels élevés de fonctionnement à la cathode (par exemple, un potentiel supérieur à 1 V par rapport à ERH (ERH signifiant électrode réversible à dihydrogène)) le platine dissous pouvant se retrouver soit dans l'eau formée au cours du fonctionnement de la pile soit à l'intérieur de la membrane électrolytique, généralement, polymérique, ce qui conduit, en son sein, à la formation de nanocristaux de platine inactifs.

Quant aux phénomènes d'augmentation, ils peuvent se produire avec des piles dont les nanoparticules de platine présentent une mobilité importante à la surface du support généralement carboné, sur lequel elles sont déposées, cette mobilité dépendant de l'énergie de surface de celui-ci.

Pour contourner ces phénomènes, il peut être fait appel à de forts taux de chargement en particules de platine avec les inconvénients que cela représente en termes de coûts de production, eu égard au prix très élevé du platine sur les marchés.

Afin de diminuer les taux de chargement tout en accédant à une surface active efficace, les études ont porté sur l'optimisation des assemblages électrode (ici, comprenant des particules de platine)-membrane.

Ainsi, il a été proposé de juxtaposer, par contact intime, les différents éléments (particules de platine, conducteur électrique et électrolyte) nécessaires à la création des points triples, cette juxtaposition pouvant consister :
- à mélanger des particules de platine avec de la poudre de carbone (remplissant le rôle de conducteur électrique) et à imprégner l'ensemble avec de l'électrolyte, de sorte à garantir un meilleur contact avec la membrane ;
- à déposer par des techniques de dépôts de couches minces (telles que l'électrodéposition ou la pulvérisation par voie physique) des particules de platine, ce qui permet de déposer du platine selon de faibles concentrations tout en conservant une activité catalytique très élevée.

Toutefois, les ensembles résultant de ces techniques sont fragiles en raison des liaisons faibles impliquées pour juxtaposer les éléments constitutifs de ces ensembles, ce qui ne permet pas d'empêcher les phénomènes de dégradation dus à la migration des particules de platine occasionnant, de ce fait, une diminution de la durée de vie de ces ensembles.

Pour pallier ces inconvénients, les auteurs de la présente invention ont proposé, dans WO 2013/068319, un procédé de préparation de particules de catalyseur, plus spécifiquement, en platine, greffées, de manière covalente, par des polymères conducteurs de protons *via* un reste organique d'un composé organique amorceur d'une polymérisation du type ATRP, des greffons spécifiques décrits dans ce document, étant des greffons de formule (l') suivante : 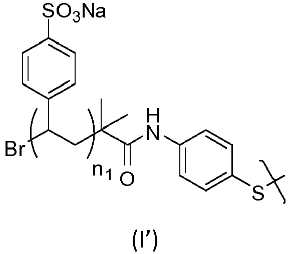 dans laquelle n₁ correspond au nombre de répétition du motif répétitif pris entre parenthèses et l'accolade indiquant l'endroit par lequel lesdits greffons sont liés de manière covalente aux particules.

Par ailleurs, Chemistry of Materials, vol.25, n°19, p.3797-3807 décrit, de manière générale, un procédé de préparation de particules comprenant un matériau apte à catalyser la réduction de l'oxygène ou l'oxydation de l'hydrogène (par exemple, des particules de platine), lesdites particules étant fonctionnalisées par des polymères comprenant au moins un motif répétitif porteur d'au moins un groupe conducteur de protons, ledit procédé comprenant successivement les étapes suivantes :
a) une étape de mise en contact de particules comprenant un matériau apte à catalyser la réduction de l'oxygène ou l'oxydation de l'hydrogène avec un composé amorceur d'une polymérisation du type ATRP, lequel composé comprend au moins un groupe apte à se greffer à la surface desdites particules, moyennant quoi l'on obtient des particules greffées par un reste dudit composé amorceur ;
b) une étape de mise en contact desdites particules obtenues en a) avec au moins un monomère porteur d'au moins un groupe conducteur de protons, moyennant quoi il y a polymérisation dudit monomère à partir des restes susmentionnés.

De manière plus spécifique, le composé amorceur d'une polymérisation du type ATRP est décrit comme pouvant être un composé comportant un groupe disulfure -S-S- formant pont entre deux parties dudit composé, au moins une de ces deux parties comportant un groupe phényle porteur d'un groupe amide -NH-CO-R¹, R¹ étant un groupe hydrocarboné porteur d'au moins un atome d'halogène (il s'entend, dans ce cas, que le groupe amide est lié à un atome de carbone du groupe phényle *via* l'atome d'azote du groupe -NH-) et plus spécifiquement un composé de formule particulière suivante :

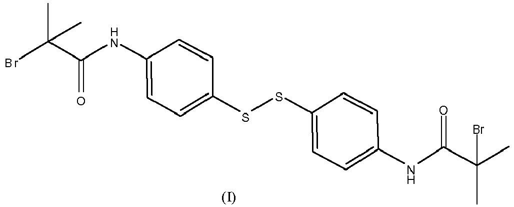

Journal of Power sources, vol. 214, p. 33-39, décrit un procédé de préparation de particules de platine liées à un matériau carboné par une opération de chauffage sous micro-ondes d'un mélange comprenant un sel de platine, un matériau carboné et un composé polyol. En partant du procédé de WO 2013/068319 , les auteurs de la présente invention se sont fixé pour objectif d'améliorer encore le procédé susmentionné et de proposer un nouveau procédé qui permettrait, en outre, d'obtenir des particules qui, une fois incorporées, dans une pile à combustible, permettraient d'obtenir une amélioration des propriétés électrochimiques de la pile à combustible, et plus spécifiquement, qui permettrait d'obtenir une bonne surface active, sans que celle-ci ne soit amoindrie par le greffage de polymères conducteurs de protons.

Aussi, les auteurs de la présente invention ont découvert qu'en utilisant un amorceur ATRP spécifique dans le cadre du procédé de l'invention ainsi qu'une méthode de synthèse spécifique des particules avant greffage, il est ainsi possible d'obtenir des particules présentant les propriétés susmentionnées.

### EXPOSÉ DE L'INVENTION

Ainsi, l'invention a trait à un procédé de préparation de particules de platine liées à un matériau carboné, lesdites particules étant greffées par des greffons consistant en au moins un polymère comprenant au moins un motif répétitif styrénique porteur d'au moins un groupe conducteur de protons, ledit procédé comprenant :
a) une étape de préparation desdites particules de platine liées à un matériau carboné comprenant une opération de chauffage sous micro-ondes d'un mélange comprenant un sel de platine, ledit matériau carboné et au moins un composé polyol, moyennent quoi sont obtenues lesdites particules ;
b) une étape de préparation d'au moins un polymère éthylénique par polymérisation ATRP d'un monomère éthylénique avec un amorceur ATRP répondant à la formule (I) suivante : 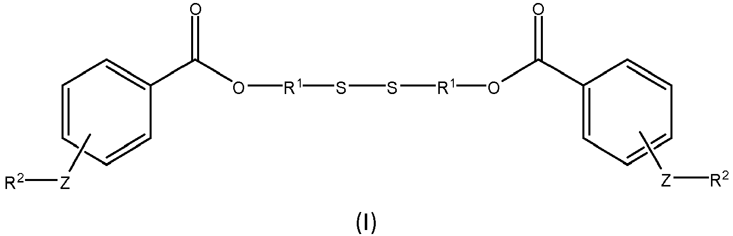 dans laquelle :
   - les groupes R¹ représentent, indépendamment l'un de l'autre, un groupe espaceur organique ;
   - les groupes Z représentent, indépendamment l'un de l'autre, une liaison simple ou un groupe espaceur organique ;
   - les groupes R² représentent, indépendamment l'un de l'autre, un atome d'halogène ;
   le polymère résultant répondant à la formule (II) suivante : 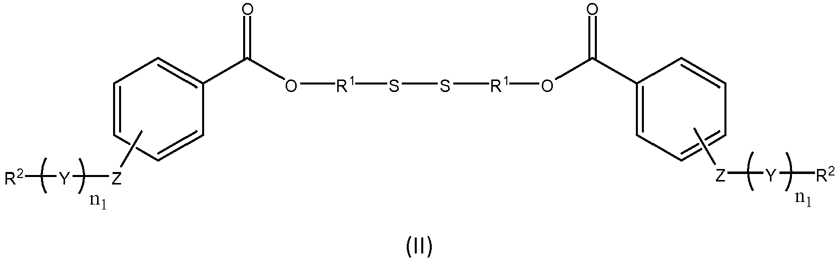 dans laquelle Y correspond au motif répétitif styrénique porteur d'au moins un groupe conducteur de protons et n₁ au nombre de répétition du motif répétitif pris entre parenthèses, les R¹, R² et Z étant tels que définis ci-dessus ;
c) une étape de mise en contact des particules obtenues en a) avec le polymère obtenu en b), moyennant quoi l'on obtient des particules greffées par des greffons de formule (III) suivante : 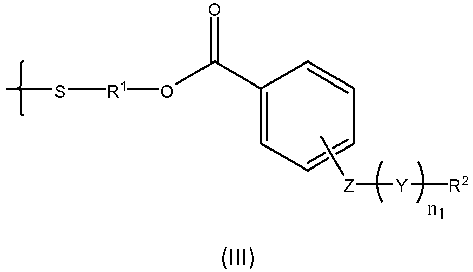 l'accolade indiquant l'endroit par lequel les greffons sont liés, de manière covalente, aux particules et les R¹, R², Z, Y et n₁ étant tels que définis ci-dessus.

En particulier, les groupes R¹ peuvent être identiques entre eux, de même que les groupes Z peuvent être identiques entre eux et les groupes R² peuvent être identiques entre eux.

Avant d'entrer plus en détail dans la présente description, nous précisons les définitions suivantes.

Par polymère, on entend, classiquement, au sens de l'invention, un composé constitué par l'enchaînement d'un ou plusieurs motifs répétitifs.

Par motif répétitif, on entend, classiquement, au sens de l'invention, un groupe organique bivalent (c'est-à-dire un groupe formant pont) issu d'un monomère après polymérisation de celui-ci.

Par polymérisation du type ATRP, on entend, une polymérisation radicalaire par transfert d'atomes (ATRP correspondant à l'abréviation de la terminologie anglaise *« Atom Transfer Radical Polymerization* »). Le mécanisme de ce type de polymérisation sera explicité plus en détail ci-dessous.

Ainsi, grâce à la mise en œuvre du procédé de l'invention, il est ainsi possible d'obtenir des particules de platine greffées par covalence *via* un reste du composé amorceur ATRP par des polymères conducteurs de protons, ce qui permet, lorsque ces particules sont destinées à entrer dans la constitution d'électrodes (notamment au niveau de couches catalytiques de celles-ci) d'assurer une bonne continuité physique avec l'électrolyte adjacent, lorsque celui-ci est à base également de polymère(s) conducteur(s) de protons.

Qui plus est, grâce au choix spécifique d'amorceur ATRP, il a été possible d'obtenir des particules, qui confèrent, aux piles, dans lesquelles elles sont incorporées, des propriétés améliorées.

Enfin, grâce à l'association de la méthode de synthèse de l'étape a) et de l'utilisation de l'amorceur spécifique ARTP susmentionné, les particules obtenues par le procédé de l'invention présentent une grande surface active et, à minimum, une conservation de celle-ci, après greffage des particules obtenues à l'étape a) voire même une amélioration de la surface active après greffage.

En d'autres termes, les auteurs de la présente invention ont été capables de développer un procédé de synthèse de particules de platine greffées par des polymères conducteurs de protons, dont la surface active est exacerbée, malgré le phénomène d'écrantage de la surface normalement inhérent au greffage de ces polymères. Aussi, grâce à la synergie obtenue par la mise en œuvre des moyens du procédé de l'invention, le greffage de ces polymères permet de rendre accessible des zones catalytiques qui ne le sont pas pour des particules non greffées.

Comme mentionné ci-dessus, le procédé de l'invention comprend, en premier lieu, une étape a) de préparation desdites particules de platine liées à un matériau carboné comprenant une opération de chauffage sous micro-ondes d'un mélange comprenant un sel de platine, ledit matériau carboné et au moins un composé polyol, moyennent quoi sont obtenues lesdites particules.

Le sel de platine peut être un sel d'halogénure de platine, éventuellement hydraté, tel que H₂PtCl₆.6H₂O.

Le matériau carboné (pouvant être assimilé à un support carboné) peut être du graphite, du noir de carbone, des fibres de carbone, des tubes de carbone (tels que des nanotubes de carbone) ou du graphène et, plus spécifiquement, du noir de carbone.

Le composé polyol, comme son nom l'indique, est un composé comprenant au moins deux groupes -OH. Il peut s'agir, en particulier, d'un composé hydrocarboné comprenant au moins deux atomes de carbone porteurs chacun d'au moins un groupe -OH, des composés correspondant à cette définition pouvant être l'éthylèneglycol ou le glycérol.

D'un point de vue pratique, le composé polyol permet la réduction du sel de platine en platine au degré d'oxydation 0 (soit, en d'autres termes, en platine métallique).

Sans être lié par la théorie, si l'on prend, pour exemple de composé polyol, l'éthylèneglycol, celui-ci assure, dans un premier temps, le rôle de solvant. Une fois le sel de platine en solution, des composés intermédiaires sont créés, tels que des composés hydroxydes et/ou oxydes. Ensuite, la déshydratation, concomitamment au chauffage, de l'éthylèneglycol en acétaldéhyde permet la réduction des ions métalliques. Cette réduction douce permet une croissance lente des particules, l'arrêt de croissance de celles-ci résultant d'une combinaison entre l'adsorption de sous-produits de la réaction sur le platine métallique (tels que les glycolates) et de l'appauvrissement du milieu en espèces à réduire (en l'occurrence, le sel de platine). Les glycolates peuvent assurer également le rôle de tensioactif qui vont s'adsorber préférentiellement sur certaines parties des particules de platine, ce qui contribue à la création d'un nombre relativement important de domaines étendus à la surface des particules.

Avantageusement, cette étape a) peut être réalisée à un pH basique, c'est-à-dire à un pH supérieur à 7, tel qu'un pH de 11, ce pH contribuant favorablement à la décomposition du sel et à la formation d'espèces, comme le glycolate, lorsque le composé polyol est l'éthylèneglycol.

Avantageusement, cette étape a) est réalisée sous atmosphère inerte, par exemple, une atmosphère d'azote.

Le chauffage sous micro-ondes peut être réalisé au moyen d'un four micro-ondes, par exemple, du type MARSXpress de la marque CEM®.

Ce chauffage peut se dérouler par une montée en température allant de la température ambiante (par exemple, 20°C) à une température de consigne (par exemple 100°C) à pression atmosphérique et une puissance d'au moins 1600 W suivie du maintien de la température de consigne à cette même puissance jusqu'à obtention des particules (par exemple, une durée de 5 minutes).

A l'issue de cette étape a), le pH du mélange obtenu est, avantageusement, ramené à un pH acide, par ajout d'une solution acide, telle qu'une solution d'acide chlorhydrique.

A l'issue de cette étape a), après l'éventuelle étape d'ajout d'une solution acide, les particules obtenues sont isolées, par exemple, par filtration (comme l'ultrafiltration), éventuellement rincées puis séchées, de sorte à éliminer les traces d'eau et de solvant.

Avant l'opération de chauffage en tant que telle, il peut être procédé à la préparation du mélange comprenant un sel de platine, un matériau carboné et au moins un composé polyol, cette préparation pouvant consister à mettre en contact ces différents ingrédients et à les mélanger, par exemple, sous ultrasons, pour obtenir, notamment, une bonne dispersion du matériau carboné.

Ensuite, le procédé de l'invention comporte une étape b) de préparation d'au moins un polymère styrénique par polymérisation ATRP d'un monomère styrénique avec un amorceur ATRP de formule (I) susmentionnée.

Cette étape de préparation est régie par les mécanismes de la polymérisation ATRP, qui fonctionne sur le principe de la formation réversible et rapide d'espèces dites « espèces dormantes » par création d'une liaison covalente avec une espèce radicalaire réactive.

Le composé amorceur d'une polymérisation du type ATRP de formule (I) est un composé comprenant au moins un groupe apte à amorcer la polymérisation ATRP, c'est-à-dire un groupe apte à se cliver au niveau d'une liaison pour former une première espèce radicalaire et une deuxième espèce radicalaire, la première espèce radicalaire réagissant ultérieurement, avec un premier carbone porteur d'une double liaison appartenant au monomère, la deuxième espèce radicalaire se fixant à un deuxième atome opposé au premier carbone porteur de la double liaison.

En d'autres termes, ce mécanisme peut être résumé selon le schéma réactionnel suivant :

X¹-X² + C=C → X¹-C-C-X²

X¹-X² correspondant à l'amorceur susmentionné avec X¹ correspondant à la première espèce et X² correspondant à la deuxième espèce, l'espèce X¹-C-C-X² étant une espèce dormante, qui peut croître par additions successives de monomères sur des radicaux libres, comme dans une polymérisation radicalaire classique, les radicaux libres étant créés par départ du groupe X², qui se fixe ensuite après insertion du monomère à l'extrémité de la chaîne polymérique, laquelle constitue toujours une espèce dormante qui peut continuer à croître dès lors qu'il subsiste des monomères dans le milieu de polymérisation.

Pour des raisons de simplicité, nous avons représenté ci-dessus uniquement la double liaison du monomère.

En outre, le composé amorceur de formule (I) utilisé dans le cadre de cette étape de préparation comprend au moins un groupe apte à se greffer à la surface des particules susmentionnées, c'est-à-dire un groupe apte à réagir avec la surface desdites particules pour former une liaison covalente, moyennant quoi il subsiste un reste de cet amorceur lié de façon covalente à la surface desdites particules.

Pour les composés amorceurs de formule (I), le groupe apte à amorcer une polymérisation du type ATRP est le groupe -Z-R² mentionné ci-dessus, ce groupe pouvant se cliver, de façon homolytique, au niveau de la liaison carbone-halogène pour former deux espèces radicalaires, une première espèce radicalaire carbone (pouvant être symbolisé par -C˙) et une deuxième espèce radicalaire consistant en un radical halogène (pouvant être symbolisé par R₂˙), la première espèce réagissant avec une extrémité de la double liaison du monomère et la deuxième espèce réagissant avec l'extrémité opposée de la double liaison. Sur la formule (I), ce groupe -Z-R² étant représenté comme entrecoupant une liaison carbone-carbone du groupe phényle, cela signifie qu'il peut être lié à l'un quelconque des atomes de carbone de ce groupe phényle.

Le groupe apte à se greffer à la surface de particules correspond, pour ce type de composés, au groupe disulfure -S-S-.

Pour les composés de formule (I), les groupes R¹ et les groupes Z peuvent représenter, indépendamment l'un de l'autre, un groupe alkylène, par exemple, un groupe éthylène, un groupe méthylène.

Plus spécifiquement, les R¹ peuvent être un groupe éthylène et les Z peuvent être un groupe méthylène.

Quand les Z représentent une liaison simple, cela signifie, en d'autres termes, que R² est directement lié à l'un quelconque des atomes de carbone du groupe phényle.

Pour les composés de formule (I), les groupes -Z-R² peuvent être situés en position *para* par rapport aux groupes -COO-.

Un composé amorceur ATRP particulier entrant dans catégorie des composés de formule (I) est un composé de formule (IV) suivante :

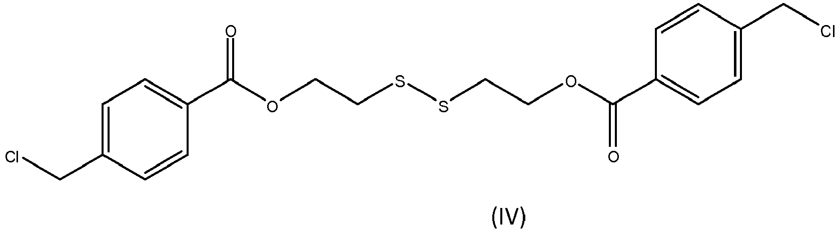

Les composés de formule (I) peuvent être synthétisés par une réaction de substitution nucléophile entre un composé halogénure d'acyle et un composé alcoolique, cette réaction étant basée sur la formation d'un alcoolate à partir de la déprotonation du composé alcoolique en milieu basique (par exemple, en présence, de triéthylamine), l'alcoolate ainsi formé réagissant sur le chlorure d'acyle pour former le composé amorceur, généralement à une température de l'ordre de 0°C, de sorte à assurer la stabilité de l'alcoolate.

A titre d'exemple, lorsqu'il s'agit de préparer un composé de formule (IV) susmentionnée, la réaction d'acylation peut se produire entre le composé 2-hydroxyéthyldisulfure et le composé chlorure de 4-chlorométhylbenzoyle selon le schéma réactionnel suivant :

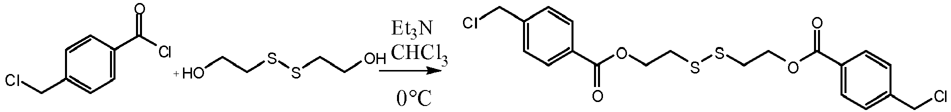

cette réaction pouvant être réalisée avec du chloroforme comme solvant organique.

Les monomères aptes à être utilisés dans le cadre de l'étape de polymérisation sont des monomères styréniques et peuvent être, plus spécifiquement, des monomères répondant à la formule (V) suivante : 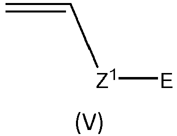 dans laquelle :
- Z¹ correspond à un groupe phénylène ; et
- E correspond à un groupe conducteur de protons, éventuellement sous forme d'un sel, tel qu'un groupe acide sulfonique, un groupe acide phosphonique ou un groupe acide carboxylique.

Un monomère spécifique répondant à la définition donnée ci-dessus est un monomère acide styrènesulfonique, par exemple sous forme d'un sel, tel qu'un sel de sodium (auquel cas, on pourra parler de monomère styrènesulfonate de sodium).

Un exemple de ce type de monomère est un monomère de formule (VI) suivante : 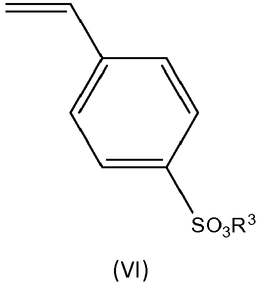 dans laquelle R³ est un atome d'hydrogène ou un cation (par exemple, un cation de métal alcalin).

Outre la présence d'un ou plusieurs monomères tels que définis ci-dessus, l'étape de polymérisation se déroule, classiquement, en présence d'un sel métallique (par exemple, un halogénure métallique, tel qu'un halogénure de cuivre, comme le chlorure de cuivre) et d'un ligand organique.

On précise que, par ligand organique, on entend un composé organique comprenant au moins un doublet libre apte à venir combler une lacune électronique d'un élément métallique (en l'occurrence, dans notre cas, une lacune électronique sur l'élément métallique du sel susmentionné) pour former un complexe métallique.

A titre d'exemple, un ligand organique approprié peut être un composé appartenant à la famille des composés pyridines, tels que la bipyridine.

L'étape de polymérisation peut être réalisée, en outre, dans un mélange eau/solvant organique (par exemple, un solvant alcoolique) sous flux d'un gaz inerte (tel qu'un flux d'argon) pendant une température et durée appropriées pour engendrer la polymérisation.

En outre, cette étape de polymérisation peut être suivie d'une étape d'hydrolyse destinée à protoner les groupes conducteurs de protons, lorsqu'ils se présentent sous forme d'un sel (soit, en d'autres termes, cette étape consiste à remplacer les cations du sel par des atomes d'hydrogène).

Les masses molaires moyennes des polymères obtenus à l'issue de l'étape de polymérisation peuvent aller de 3 000 à 1 000 000 g/mol et plus spécifiquement de 80 000 à 700 000 g/mol et, plus spécifiquement encore, de 300 000 à 600 000 g/mol.

Après l'étape b), le procédé de l'invention comprend une étape c) de mise en contact des particules obtenues en a) avec le polymère obtenu en b), moyennant quoi l'on obtient des particules greffées par des greffons de formule (III) suivante : 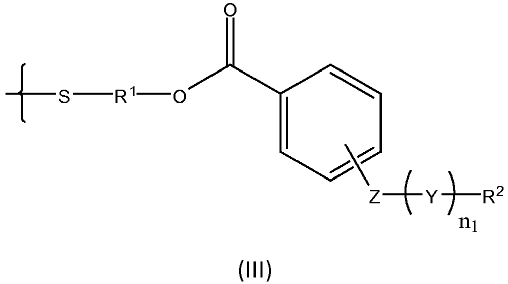 le groupe -Z-(Y)ₙ₁-R², qui entrecoupe une liaison carbone-carbone du groupe phényle, signifiant qu'il peut être lié à l'un quelconque des atomes de carbone du groupe phényle.

Cette étape c) de mise en contact peut comprendre une opération de dispersion des particules susmentionnées, par exemple, dans un solvant électrophile (tel qu'un solvant amine, tel que l'hexylamine) suivie d'une opération de mise en contact de la dispersion obtenue avec un ou plusieurs polymères tels que définis ci-dessus dans des conditions suffisantes pour permettre le greffage par covalence des polymères préparés en b).

Sans être lié par la théorie, le polymère, en présence de particules, va se scinder en deux restes organiques par clivage homolytique de la liaison entre les deux atomes de soufre, les deux restes consistant en des espèces radicalaires comportant des électrons libres situés au niveau des atomes de soufre, ces électrons libres s'associant chacun avec un électron présent à la surface des particules pour former une liaison covalente entre les restes susmentionnés et les particules *via* les atomes de soufre, le produit résultant pouvant être schématisé de la façon suivante : 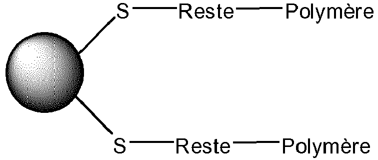 la sphère pleine correspondant à une particule, -S-Reste- correspondant à un reste du composé amorceur ATRP formant pont entre la particule et le polymère (respectivement, une première chaîne polymérique et une deuxième chaîne polymérique).

Concernant le polymère, le groupe conducteur de protons peut être un groupe acide sulfonique -SO₃H, un groupe acide carboxylique -CO₂H ou un groupe acide phosphonique -PO₃H₂, ces groupes pouvant être présents éventuellement sous forme de sels.

A l'issue du procédé de l'invention, le taux de greffage de polymère(s) (exprimé en pourcentage massique de polymère dans les particules) peut aller de 0,01 à 30% massique et, plus spécifiquement allant de 0,5 à 15% massique et, plus spécifiquement encore, allant de 1 à 5% massique, ce dernière gamme étant idéalement associée à des polymères présentant une masse molaire moyenne allant de 300 000 à 600 000 g/mol.

Les particules susceptibles d'être obtenues par le procédé de l'invention, sont des particules de platine liées à un matériau carboné (par exemple, de manière covalente), lesdites particules étant greffées par des greffons de formule (III) suivante : 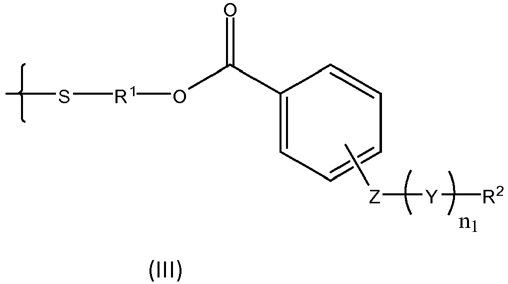 dans laquelle les R¹, R², Z, Y et n₁ répondent aux mêmes définitions que celles données ci-dessus, le groupe -Z-(Y)ₙ₁-R², qui entrecoupe une liaison carbone-carbone du groupe phényle signifiant qu'il peut être lié à l'un quelconque des atomes de carbone du groupe phényle.

Quant au matériau carboné, il peut être du graphite, du noir de carbone, des fibres de carbone, des tubes de carbone (tels que des nanotubes de carbone), du graphène.

Le rapport massique entre matériau carboné et platine peut être compris entre 80/20 et 20/80, idéalement entre 45/55 et 65/35.

Plus spécifiquement, R¹ et Z représentent un groupe alkylène et R² un atome d'halogène, tel que du chlore, tandis que Y peut représenter un motif répétitif issu de la polymérisation d'un monomère styrénique de formule (V) ou (VI) susmentionnée, tel qu'un monomère styrènesulfonate de sodium. Le groupe -Z-(Y)ₙ₁-R² peut être également en position *para* par rapport au groupe -CO-O-.

Pour information, l'accolade indique l'endroit par lequel les greffons sont liés, de manière covalente, aux particules, elles-mêmes étant liées à un matériau carboné.

Des particules spécifiques conformes à l'invention peuvent être des particules de platine greffées par des greffons de formule (VII) suivante : 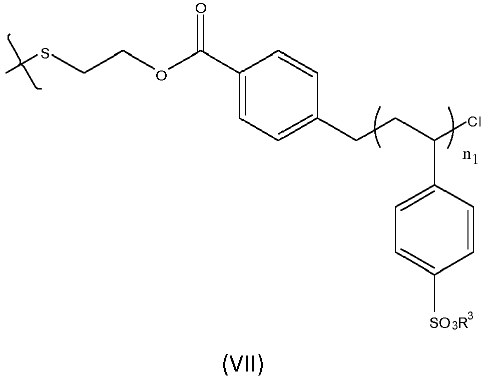 avec R³ et n₁ étant tel défini ci-dessus.

De telles particules sont particulièrement intéressantes, car elles permettent de transposer la phénoménologie du point triple à l'échelle moléculaire, le rôle du catalyseur étant rempli par le matériau constitutif de la particule en tant que telle, le rôle du conducteur protonique étant rempli par les polymères susmentionnés et le rôle du conducteur électronique étant rempli par le matériau carboné. Les liaisons covalentes entre le conducteur électronique et le catalyseur d'une part et entre le matériau conducteur protonique et le catalyseur d'autre part assurent, premièrement, un meilleur transfert des charges (respectivement, électrons et protons) et donc de meilleures performances et, deuxièmement, une parfaite stabilité en conditions de fonctionnement en pile, lorsque ces particules sont utilisées dans des piles. Ces deux résultats permettent de réduire le taux de chargement en catalyseur pour des performances accrues.

Les particules de l'invention sont aptes à catalyser la réduction de l'oxygène ou l'oxydation de l'hydrogène et présentent, qui plus est, une importante surface active.

Les particules de l'invention peuvent entrer, donc tout naturellement, dans la constitution d'électrodes de piles à combustible, en particulier de piles à combustible du type PEMFC, plus particulièrement dans des couches catalytiques d'électrodes de piles à combustible.

Ainsi, l'invention a également trait à des électrodes comprenant de telles particules et à des piles à combustible comprenant au moins un assemblage électrode-membrane-électrode, dans lequel au moins une de ses électrodes est une électrode conforme à l'invention.

Ces particules ne présentent aucun signe de dégradation en-dessous de 220°C (cette limite étant déterminée par ATG). Par ailleurs, la tenue électrochimique de la couronne organique (constituée par les polymères greffés aux particules) a été démontrée dans une gamme de potentiels de 0 à 1,2 V vs ERH (ERH signifiant électrode réversible à dihydrogène), ce qui permet d'envisager d'utiliser ces particules comme catalyseurs pour les piles à combustible du type PEMFC.

Ces particules sont dispersables dans une solution alcoolique. Il est dès lors possible de les mélanger à un ionomère conducteur protonique dans des proportions allant de 100/0 à 70/30. La solution peut alors être déposée sur tout type de support poreux carboné (tissu ou feutre), le produit résultant pouvant être utilisé comme électrode de pile à combustible.

Qui plus est, les particules de l'invention, une fois incorporées dans des piles à combustible, permettent d'obtenir une amélioration des propriétés, telles que la tension en circuit ouvert, une meilleure activation des réactions électrochimiques au sein de la pile, une chute ohmique moins importante et une meilleure puissance délivrée, par rapport à des piles à combustible similaires, dans lesquelles cependant les particules introduites sont des particules greffées par des greffons de formule (I').

Par ailleurs, ces particules présentent une activité électrocatalytique même lorsqu'elles sont mises en œuvre sans ionomère de type Nafion. Ce résultat particulièrement remarquable permet de réaliser des électrodes sans Nafion. Associées à des membranes alternatives au Nafion, ces particules permettront de réaliser des assemblages membrane/électrode libre de tout Nafion.

Ainsi, les piles à combustible, par exemple du type PEMFC, comprennent, classiquement, au moins un assemblage électrode-membrane-électrode, dans lequel au moins une de ses électrodes est à base de particules conforme à l'invention.

La membrane quant à elle peut être à base d'un matériau polymérique conducteur de protons, le ou les polymères constitutifs de ce matériau pouvant être de même nature que le ou les polymères greffés à la surface desdites particules.

L'invention va être à présent décrite, par rapport aux exemples suivants donnés à titre illustratif et non limitatif.

### BREVE DESCRIPTION DES FIGURES

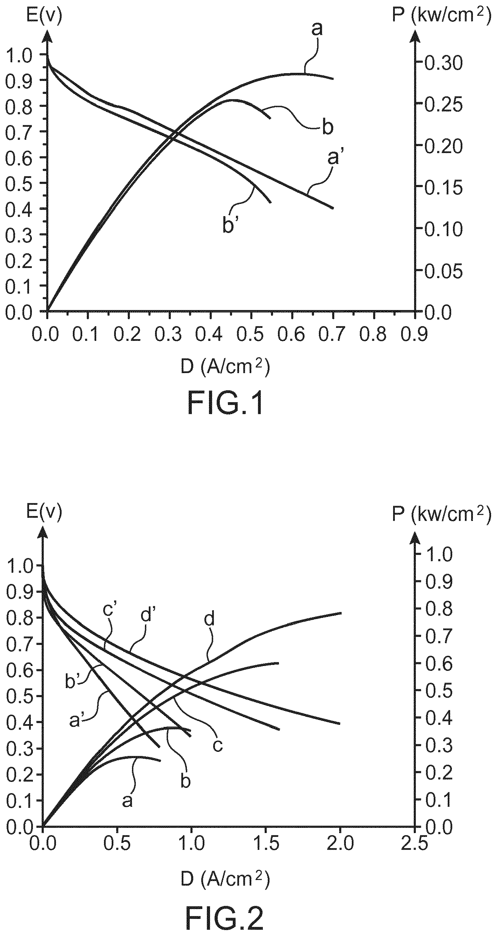

La figure 1 illustre des courbes de polarisation représentant l'évolution de la tension E (en V) en fonction de la densité de courant D (en A/cm²) pour la Pile 1 (courbe a') et la Pile 2 (courbe b') de l'exemple 5 et des courbes de puissance représentant l'évolution de la densité de puissance P (en kW/cm²) en fonction de la densité de courant D (en A/cm²) (respectivement courbes a) et b) pour les Piles 1 et 2).
La figure 2 illustre des courbes de polarisation représentant l'évolution de la tension E (en V) en fonction de la densité de courant D (en A/cm²) (respectivement courbes a'), b'), c') et d') pour des piles comprenant des particules des essais 2, 3, 4 et 7) et les courbes de puissance représentant l'évolution de densité de puissance P (en kW/cm²) en fonction de la densité de courant D (en A/cm²) (respectivement courbes a), b), c) et d) pour des piles comprenant des particules des essais 2, 3, 4 et 7).

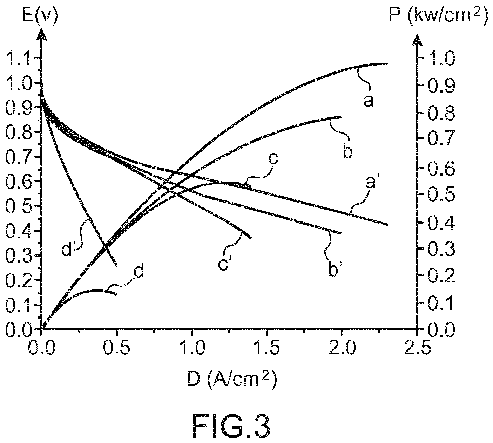

La figure 3 illustre des courbes de polarisation représentant l'évolution de la tension E (en V) en fonction de la densité de courant D (en A/cm²) (respectivement courbes a'), b'), c') et d') pour des piles comprenant des particules des essais 6, 7, 8 et 9) et les courbes de puissance représentant l'évolution de densité de puissance P (en kW/cm²) en fonction de la densité de courant D (en A/cm²) (respectivement courbes a), b), c) et d) pour des piles comprenant des particules des essais 6, 7, 8 et 9).

### EXPOSÉ DÉTAILLÉ DE MODES DE RÉALISATION PARTICULIERS

### EXEMPLE 1

Cet exemple illustre la préparation d'un amorceur ATRP conforme à l'invention : le disulfanediyldiéthane-2,1-diyl bis[4-(chlorométhyl)benzoate] de formule (IV) suivante :

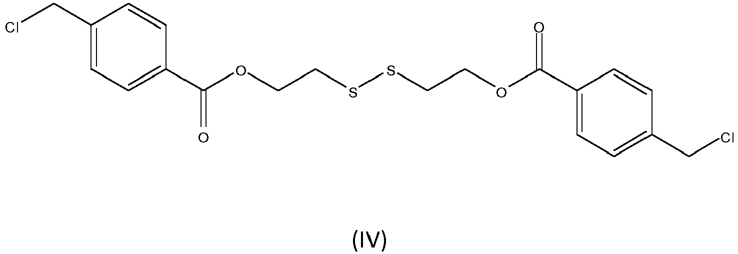

Pour ce faire, dans un bicol de 100 mL sont introduits, sous atmosphère inerte, du 2-hydroxyéthyldisulfide (1,53 g ; 9,9 mmol ; 1 éq.), du chloroforme (30 mL) et de la triéthylamine (4,22 g ; 41,7 mmol ; 4,2 éq.). Le bicol est scellé sous argon puis plongé dans un bain de glace à 0°C.

Ensuite, du chlorure de 4-chlorométhylbenzoyle (2,06 g ; 10,9 mmol ; 1,1 éq.) est introduit au goutte-à-goutte. Puis on laisse le mélange revenir à température ambiante pendant une nuit. Le mélange réactionnel résultant est lavé 4 fois (un lavage acide, un lavage neutre, un lavage basique puis un lavage neutre). Les phases organiques sont réunies et séchées. Le solvant organique est ensuite éliminé à l'évaporateur rotatif. Le produit solide obtenu est ensuite séché à l'étuve à 60°C pendant une nuit.

Le produit résultant (avec un rendement de 97%) correspond au produit attendu de formule ci-dessus selon les analyses de spectroscopie RMN ¹H et d'analyse élémentaire, dont les résultats figurent ci-dessous.
**RMN ¹H** (400 MHz, CDCl₃, 6=7,26 ppm) : 8,5 (s, 1H, NH); 7,5-7,4 (m, 4H, H aromatique); 2,0 (s, 6H, CH₃)
**RMN** ¹³**C** (100 MHz, CDCl₃, δ=77,0 ppm): 170 (s, C=O); 137,2 (s, C_{q}-S); 132,8 (s, C_{q}-NH); 130,1 (d, HN-C_{q}-CH-CH-S); 120,5 (d, HN-, C_{q}-CH-CH-S); 63,0 (s, Br-C(CH₃)₂); 32,5 (q, CH₃)
**Analyse élémentaire (en %) :** (C₂₀H₂₀Cl₂O₄S₂), C: 52,1 ; H: 4,4; Cl:15,5 ; O: 14; S: 14.

### EXEMPLE 2

Cet exemple illustre la préparation d'un polymère pouvant être schématisé par la formule suivante ci-dessous : 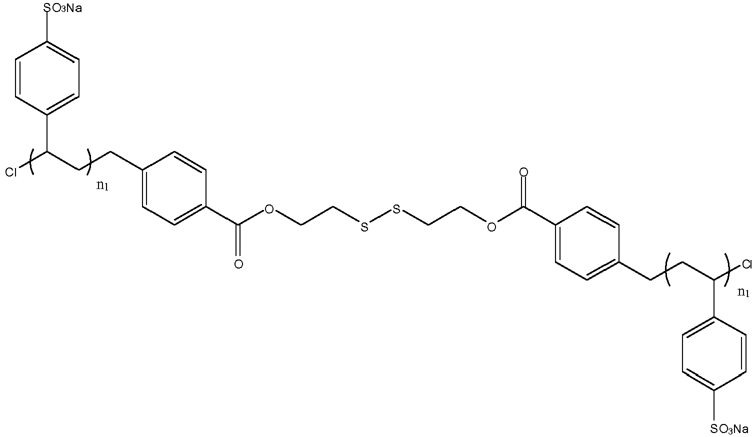 avec n₁ correspondant au nombre de répétition du motif pris entre parenthèses.

Différents essais ont été mis en œuvre avec des quantités de monomères différentes (X).

Pour ce faire, dans un premier temps, un bicol de 100 mL est soumis à un traitement thermique sous vide comprenant 3 cycles avec une phase de chauffage et une phase de refroidissement à température ambiante.

Puis de l'eau MilliQ (48 mL) est introduite dans le bicol et est dégazée sous vide en faisant buller de l'argon (15 minutes). Du styrènesulfonate de sodium (X g ; Y mol ; Z éq.) est ensuite introduit sous flux d'argon et de l'argon est remis à buller sous vide.

En parallèle, du méthanol (16 mL) est dégazé sous vide en faisant buller de l'argon (15 minutes) dans un ballon piriforme de 25 mL. L'amorceur préparé à l'exemple 1 (50 mg ; 0,09 mmol ; 1 éq.) est ensuite introduit sous flux d'argon.

Lorsque le monomère est parfaitement dissous dans l'eau, de la bipyridine (116 mg ; 0,74 mmol ; 8 éq.) et du chlorure de cuivre (37 mg ; 0,37 mmol ; 4 éq.) sont introduits sous flux d'argon.

De l'argon est mis à buller dans le système tout en tirant sous vide.

La solution d'amorceur dans le méthanol est introduite avec une seringue (20 mL) en veillant à conditionner celle-ci sous argon. Trois cycles vide-argon sont enfin effectués.

Le bicol est enfin mis en place dans un bain d'huile préalablement chauffé à 45°C. Après environ, 21 heures de polymérisation, la réaction est stoppée par mise à l'air du système. La solution passe d'une couleur marron à un couleur verte-bleue.

Le mélange réactionnel est ensuite filtré sur gel de silice, afin d'éliminer les ions chlorure contenus dans le système catalytique et piégés dans le polymère.

Le filtrat est ensuite concentré sous vide, afin d'augmenter la concentration en polymère et donc de faciliter la précipitation.

Enfin, le polymère est précipité dans du méthanol froid.

Le polymère obtenu est un solide blanchâtre pégueux puis est placé à l'étuve à 65°C pendant 1 nuit.

Le polymère résultant correspond au produit attendu de formule ci-dessus selon les analyses RMN ¹H, dont les résultats figurent ci-dessous.

**RMN ¹H** (D₂O) δ: 7,5 (s large, proton aromatique), 6,6 (s large, proton aromatique), 1,4 (s large, proton méthylique).

Les quantités de monomères utilisées sont récapitulées dans le tableau suivant :

| Degré de polymérisation visé (DP) | Masse monomère (g) X | Nombre de moles Y (en moles) | Nombre d'équivalents Z |
|---|---|---|---|
| 500 | 9,4 | 0,045 | 500 |
| 1000 | 18,8 | 0,09 | 1000 |
| 1500 | 28,2 | 0,135 | 1500 |
| 2000 | 37,6 | 0,18 | 2000 |
| 2500 | 47 | 0,225 | 2500 |

Les caractéristiques des polymères obtenus pour chacun des essais sont reportés dans le tableau suivant.

| Degré de polymérisation visé (DP) | Masse molaire du polymère obtenu (en g) | Degré de polymérisation expérimental |
|---|---|---|
| 500 | 76 000 | 370 |
| 1000 | 140 000 | 680 |
| 1500 | 222 000 | 1080 |
| 2000 | 300 000 | 1460 |
| 2500 | 359 000 | 1740 |

### EXEMPLE 3

Cet exemple illustre la préparation de particules de platine liées à un matériau carboné du type noir de carbone (dénommé, dans la formule ci-dessous "Vulcan XC72"), réprésentées par la formule ci-dessous: 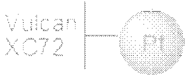 selon deux méthodes:
- une méthode non conforme à l'invention impliquant une microémulstion dite "eau-dans-huile" (dite méthode 3a); et
- une méthode conforme à l'invention impliquant un milieu polyol et un chauffage par irradiation micro-onde (dite méthode 3b).

### a) Synthèse des particules par microémulsion dite "eau-dans-huile"

Dans un réacteur, on verse de l'heptane (37,4 g) et du Brij® 30 (8,6 g). Le réacteur est ensuite agité manuellement et immédiatement de manière vigoureuse, afin d'éviter la précipitation du Brij® 30, jusqu'à ce que le mélange soit bien translucide.

En parallèle, un sel de platine hexahydraté H₂PtCl₆.6H₂O (257,6 mg ; 0,5 mmol, 1 éq.) est dissous dans 2 mL d'eau milliQ dans un pilulier. La solution est bien agitée, jusqu'à ce que la solution soit bien homogène.

On ajoute, dans le réacteur susmentionné, 1,6 mL de la solution de sel métallique puis on agite manuellement, jusqu'à ce que le mélange soit limpide. Ce mélange prèsente une couleur jaune orangée.

Le mélange résultant est laissé au repos pendant une durée de 15 minutes, afin qu'il se stabilise.

Du borohydrure de sodium (152 mg ; 4 mmol; 15 éq.) est ajouté au mélange en une seule fois rapidement et le mélange est agité immédiatement de manière vigoureuse (le borohydrure de sodium devant réduire le métal avant l'eau). Le mélange passe à une coloration noire intense.

Le mélange est laissé au repos pendant une durée de 1 à 2 heures, de manière à ce que le platine soit totalement réduit et que le NaBH₄ soit totalement désactivé.

Après deux heures, le mélange est placé sous ultrasons pendant 5 minutes, en agitant de temps en temps, de manière à ce qu'il n'y ait plus de dépôt au fond du réacteur.

Du noir de carbone Vulcan®XC 72 (préalablement finement broyé) est ajouté dans le mélange, pendant que celui-ci est toujours soumis aux ultrasons, ceux-ci étant maintenus pendant 5 minutes, une fois l'ajout effectué, tout en maintenant une agitation sporadique. Après 5 minutes, le réacteur est agité fortement de manière manuelle puis, après vérification que le noir de carbone ne se dépose pas au fond du réacteur, celui-ci est soumis de nouveau aux ultrasons pendant 5 minutes avant d'être, encore une fois, agité manuellement. Cette manipulation est réitérée, jusqu'à ce que le carbone ne se dépose plus au fond du réacteur.

Puis, une fois le noir de carbone bien en suspension, le réacteur est laissé dans le bain à ultrasons. De l'acétone (1 volume d'acétone pour un volume de microémulsion) est ajouté petit à petit, en agitant de façon manuelle à chaque phase d'ajout. Le mélange résultant est laissé 5 à 10 minutes dans le bain à ultrasons après la fin de l'ajout.

Les particules sont ensuite isolées par ultrafiltration sur une membrane hydrophile en polyfluorure de vinylidène (PVDF) Durapore (0,22 µm; GVWP 04700) sous vide. Les particules de platine supportées sur le matériau carboné (le noir de carbone) sont lavées par filtration par cycles de 4*30 mL d'acétone, 4*30 mL d'éthanol et 4*30 mL d'eau MilliQ (avec une agitation entre chaque lavage). Les particules obtenues sont alors placées pendant 2 heures à l'étuve à une température de 135°C, pour éliminer les dernières traces de Brij® 30.

Le rendement est quantitatif.

Les particules obtenues sont analysées par analyse élémentaire attestant de la présence de carbone (à hauteur de 60%) et de platine (à hauteur de 40%), ce qui démontre que les particules de platine sont supportées sur le matériau carboné.

### b) Synthèse des particules en milieu polyol par voie micro-onde

Dans un premier temps, un sel de platine hexahydraté de formule H₂PtCl₆.6H₂O (257 mg) est dissous dans 100 mL d'éthylène glycol. Le pH est alors d'environ 0,8. Il est ajusté à 11 par ajout d'une solution de soude.

Du noir de carbone Vulcan®XC 72 (préalablement finement broyé) (0,145 mg) est ensuite ajouté à la solution précédemment obtenue et le mélange résultant est placé aux ultrasons, jusqu'à dispersion totale du noir de carbone.

Le mélange est chauffé par irradiation micro-onde dans un four à micro-ondes MARSXPress de la marque CEM® sous atmosphère inerte d'azote (Montée en température de 20°C à 100°C à pression atmosphérique et à une puissance de 1600 W ; Maintien pendant 5 minutes à 100°C à 1600 W; Pulse: 80%).

Le pH obtenu à la fin de la synthèse est égal à 11 à une température de 18°C. Le pH est ajusté à 2 par ajout d'une solution d'acide chlorhydrique, puis 50 mL d'eau milliQ sont ajoutés pour homogénéiser le mélange. Le mélange résultant est ensuite placé aux ultrasons pendant 5 minutes.

Les particules sont ensuite isolées par ultrafiltration puis rincées abondamment à l'eau milliQ et enfin séchées à 60°C à l'étuve avant d'être placées à 200°C pendant 2 heures à l'étuve.

### EXEMPLE 4

Cet exemple illustre la préparation de particules de platine préparées selon les modes de préparation de l'exemple 3 greffées par le polymère préparé à l'exemple 2, ces particules étant ainsi greffées par des greffons de formule suivante : 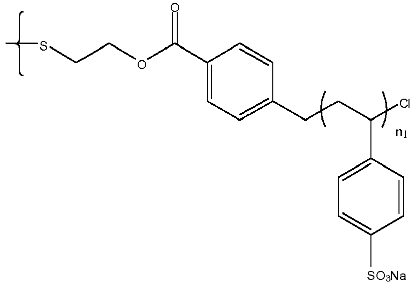 n₁ indiquant le nombre de répétition du motif pris entre parenthèses.

Que ce soient pour les particules préparées sur la méthode 3a) ou les particules préparées selon la méthode 3b), le protocole de préparation est le suivant.

Dans un ballon de 25 mL, sont introduites les particules préparées à l'exemple 3 et de l'hexylamine. Le ballon est placé 15 minutes dans un bain à ultrasons, afin que la suspension de particules soit homogène. Le polymère obtenu à l'exemple 2 est mis en solution dans un mélange eau/hexylamine (50/50 en volume) puis est introduit dans le ballon. L'ensemble est placé sous agitation magnétique pendant 12 heures.

Les particules de platine fonctionnalisées sont isolées par ultrafiltration puis subissent différents étapes en commençant par une précipitation dans l'acétone puis soumises à différentes étapes de lavage (3*30 mL d'acétone, 3*30 mL d'éthanol et 3*30 mL d'eau).

Ces étapes de lavage permettent d'éliminer les traces de polymères susceptibles de ne pas s'être greffés sur les particules.

Les particules sont ensuite placées une nuit à l'étuve à 65°C.

Le tableau ci-dessous regroupe les résultats des différents essais réalisés selon les modalités du protocole exposé ci-dessus.

| Essai | Masse molaire du polymère (g) | Quantité de polymère engagée (en mg) | Quantité de particules engagée* (en mg) | Rapport massique de polymère théorique (en %) | Rapport massique de polymère expérimental *(en %) | Nombre de moles de polymère/ g de particules |
|---|---|---|---|---|---|---|
| 1 (Méthode 3b) | 76000 | 1,7 | 100 | 1,69 | 1,69 | 5,6^{∗}10⁻⁷ |
| 2 (Méthode 3b) | 140000 | 3,1 | 100 | 3,05 | 3,05 | 5,6^{∗}10⁻⁷ |
| 3 (Méthode 3b) | 222000 | 5,0 | 100 | 4,76 | 4,76 | 5,6^{∗}10⁻⁷ |
| 4 (Méthode | 300000 | 6,8 | 100 | 6,33 | 6,33 | 5,6^{∗}10⁻⁷ |
| 3b) | | | | | | |
| 5 (Méthode 3b) | 359000 | 2,8 | 100 | 2,75 | 2,75 | 2,0^{∗}10⁻⁷ |
| 6 (Méthode 3b) | 359000 | 4,0 | 100 | 3,89 | 3,88 | 2,8^{∗}10⁻⁷ |
| 7 (Méthode 3b) | 359000 | 8,1 | 100 | 7,48 | 7,48 | 5,6^{∗}10⁻⁷ |
| 8 (Méthode 3b) | 359000 | 16,2 | 100 | 13,92 | 13,92 | 1,1^{∗}10⁻⁶ |
| 9 (Méthode 3b) | 359000 | 24,3 | 100 | 19,52 | 19,52 | 1,7^{∗}10⁻⁶ |
| 10 (Méthode 3b) | 359000 | 32,3 | 100 | 24,44 | 24,43 | 2,3^{∗}10⁻⁶ |
| 11 (Méthode 3a) | 0 | 0 | 100 | 0 | 0 | 0 |
| 12 (Méthode 3b) | 0 | 0 | 100 | 0 | 0 | 0 |
| 13 (Méthode 3a) | 140000 | 3,1 | 100 | 3,05 | 3,05 | 5,6^{∗}10⁻⁷ |

| | | | | | | |
|---|---|---|---|---|---|---|
| *mesurée par analyse élémentaire | | | | | | |

### EXEMPLE 5

Dans cet exemple, des particules obtenues à l'exemple 4 sont soumises à des analyses électrochimiques et à des tests en pile.

### a) Caractérisation électrochimique des particules

Les propriétés électrochimiques (surface accessible, activité et sélectivité) des catalyseurs sont des facteurs importants dans le choix du matériau à utiliser au sein des couches actives cathodiques des piles à combustible du type PEMFC.

La présence d'une couronne organique pourrait modifier, de manière importante ses propriétés catalytiques, qui sont, en théorie, fortement liées à l'état de surface du catalyseur.

Les résultats des analyses électrochimiques (surface active et activité catalytique) pour des particules obtenues selon les deux méthodes de synthèse de nanoparticules sont résumés dans le tableau ci-dessous.

| Particules | Surface active (en m²/g) | Activité (jₖ) (0,9 V) (en mA/cm²) |
|---|---|---|
| Essai 11 | 30 | 2,29 |
| Essai 12 | 80 | 4,59 |
| Essai 13 | 22 | 2,90 |
| Essai 2 | 67 | 4,03 |

De ce tableau, l'on peut constater qu'il y a une grande différence de surface active entre les particules obtenues par la méthode 3a) (à savoir, la méthode impliquant une microémulsion « eau-dans-huile ») et les particules obtenues par la méthode 3b (à savoir, la méthode impliquant un polyol et un chauffage par micro-ondes).

En effet, il ressort que les particules obtenues par la méthode 3b présentent une surface active environ 2,5 fois plus importante que les particules obtenues par la méthode 3a. Pour information, la surface active est déterminée par voltammétrie cyclique à variation linéaire de potentiel en milieu support (HClO₄ 0,1M, désaéré par un gaz inerte)

De plus, l'on peut constater que les particules obtenues par la méthode 3b présentent une meilleure activité (jₖ) vis-à-vis de la réaction de réduction de l'oxygène. Pour information, l'activité catalytique du catalyseur est déterminée à partir de la méthode de Koutecky-Levich appliquée aux mesures des courbes de polarisation effectuées à l'aide d'une électrode à disque tournant. Le disque tournant permet d'enregistrer la réduction de l'oxygène dans l'électrolyte liquide à différentes vitesses de rotation de l'électrode.

Les caractérisations sont réalisées dans les conditions suivantes :
- vitesses de rotation de l'électrode : 2500, 2000, 1500,1000 et 500 tr.min⁻¹ ;
- vitesse de variation linéaire de potentiel fixée à 1 mV/s ;
- milieu HClO4 0,1 M saturé en oxygène.

### b) Test en piles

Pour ce faire, il a été réalisé des piles (respectivement Pile 1 et Pile 2) comportant chacune un assemblage électrode-membrane-électrode comprenant chacun :
- une anode du type électrode à diffusion de gaz comprenant 0,2 mg/cm² de particules commerciales de platine liées à un matériau carboné (ces particules étant non greffées) ;
- une cathode du type électrode à diffusion de gaz comprenant 0,4 mg/cm² de particules de platine obtenues par l'un des essais de l'exemple 4;
- une membrane en Nafion® NRE 211 disposée entre l'anode et la cathode.

Pour la Pile 1, la cathode comprend des particules de platine obtenues à l'essai 2 de l'exemple 4.

Pour la Pile 2, la cathode comprend des particules de platine obtenues à l'essai 13 de l'exemple 4.

Les assemblages électrode-membrane-électrode sont réalisés selon le protocole opératoire suivant.

Que ce soient pour l'anode ou pour la cathode, celles-ci sont préparées par simple coulée d'une encre catalytique comprenant les particules de platine concernées dans un mélange éthanol/eau (3 :1) sur un tissu à diffusion de gaz (GDL) en Sigracet® 24BC.

Avant d'être placée dans l'assemblage, la membrane en Nafion® est traitée préalablement par pressage à chaud en venant presser un renfort de part à d'autre de celle-ci à une température de 110°C et à une pression de 3MPa pendant 90 secondes.

Enfin, les électrodes à diffusion de gaz (anode et cathode) sont pressées de part et d'autre de la membrane en Nafion® ainsi traitée préalablement à une température de 115°C et à une pression de 3,5 MPa pendant 150 secondes.

Les tests sont réalisés en monocellule de 5 cm² sous H₂/O₂ (stoechiométrie λ_{O2}=1,5 et λ_{H2}=1,5) sous une pression de 2 bars, à 60°C et à 21% d'humidité.

Des courbes d'évolution de la tension E (en V) en fonction de la densité de courant (en A/cm²) sont reportées sur la figure 1 (courbe a' pour la Pile 1 et courbe b' pour la Pile 2) ainsi que les courbes de puissance (courbe a pour la Pile 1 et courbe b pour la Pile 2).

Les tests en pile démontrent que le catalyseur préparé par la méthode de synthèse polyol par voie micro-onde présente une meilleure activité. Cette différence d'activité est observable dans la zone d'activation correspondant aux faibles densités de courant, c'est-à-dire une densité de courant comprise entre 0 et 0,1 A/cm². En effet, dans cette zone, la courbe a' présente une pente bien moins importante que celle de la courbe b', ce qui traduit une meilleure activité catalytique et ainsi un meilleur démarrage des réactions au niveau de la pile à combustible d'où une chute de tension moindre.

Il est à remarquer, également, que le catalyseur préparé par la méthode de synthèse polyol par voie micro-onde est moins impacté par les problèmes de diffusion des réactifs, ce qui se traduit par une courbe a') qui ne présente pas une allure de courbe correspondant à un noyage aux fortes densités de courant, contrairement à la courbe b'). Ce phénomène de noyage peut s'expliquer par la production d'eau importante qui fait barrage à la diffusion des réactifs.

### EXEMPLE 6

Dans cet exemple, les particules obtenues aux essais 1, 2, 3, 4 et 7 de l'exemple 4 ont été testées pour déterminer leur surface active et leur activité électrochimique vis-à-vis de la réaction de réduction de l'oxygène.

Les résultats sont reportés dans le tableau ci-dessous.

| Particules | Degré de polymérisation en nombre DPₙ | Nombre de moles de polymère/ g de particules | Surface active (en m²/g) | Activité (jₖ) (0,9 V) (en mA/cm²) |
|---|---|---|---|---|
| Essai 1 | 500 | 5,6^{∗}10⁻⁷ | 55 | 1,43 |
| Essai 2 | 1000 | 5,6^{∗}10⁻⁷ | 63 | 4,03 |
| Essai 3 | 1500 | 5,6^{∗}10⁻⁷ | 69 | 3,53 |
| Essai 4 | 2000 | 5,6^{∗}10⁻⁷ | 73 | 3,74 |
| Essai 7 | 2500 | 5,6^{∗}10⁻⁷ | 86 | 4,16 |

Ces résultats montrent des propriétés particulièrement intéressantes en termes de surface active et d'activité pour les particules obtenues par la méthode de synthèse polyol par voie micro-onde et, en particulier, celles greffées par des polymères de forte masse moléculaire.

### EXEMPLE 7

Dans cet exemple, les particules obtenues aux essais 2, 3 4 et 7 de l'exemple 4 ont été testées en pile dans les mêmes conditions d'assemblage et de test que celles explicitées à l'exemple 5 ci-dessus.

Les résultats sont reportés sur la figure 2 jointe en annexe, qui illustre, d'une part, les courbes de polarisation représentant l'évolution de la tension E (en V) en fonction de la densité de courant D (en A/cm²) (respectivement courbes a'), b'), c') et d') pour des piles comprenant des particules des essais 2, 3, 4 et 7) et les courbes de puissance représentant l'évolution de densité de puissance P (en kW/cm²) en fonction de la densité de courant D (en A/cm²) (respectivement courbes a), b), c) et d) pour des piles comprenant des particules des essais 2, 3, 4 et 7).

Il est observé des résultats particulièrement intéressants avec les particules obtenues par la méthode de synthèse polyol par voie micro-onde et, en particulier, celles greffées par des polymères de forte masse moléculaire et avec des taux de greffage de 5,6*10⁻⁷.

### EXEMPLE 8

Dans cet exemple, les particules obtenues aux essais 12, 5, 6, 7, 8, 9 et 10 de l'exemple 4 ont été testées pour déterminer leur surface active et leur activité électrochimique vis-à-vis de la réaction de réduction de l'oxygène.

Les résultats sont reportés dans le tableau ci-dessous.

| Particules | Degré de polymérisation en nombre DPₙ | Nombre de moles de polymère/ g de particules | Surface active (en m²/g) | Activité (jₖ) (0,9 V) (en mA/cm²) |
|---|---|---|---|---|
| Essai 12 | - | - | 80 | 4,59 |
| Essai 5 | 2500 | 2,0^{∗}10⁻⁷ | 89 | 3,86 |
| Essai 6 | 2500 | 2,8^{∗}10⁻⁷ | 98 | 8,63 |
| Essai 7 | 2500 | 5,6^{∗}10⁻⁷ | 86 | 4,16 |
| Essai 8 | 2500 | 1,1^{∗}10⁻⁶ | 83 | 2,93 |
| Essai 9 | 2500 | 1,7^{∗}10⁻⁶ | 73 | 1,34 |
| Essai 10 | 2500 | 2,3^{∗}10⁻⁶ | 68 | 1,31 |

Ces résultats montrent des propriétés particulièrement intéressantes en termes de surface active et d'activité pour les particules obtenues par la méthode de synthèse polyol par voie micro-onde et, en particulier, celles avec des taux de greffage intermédiaire.

De manière surprenante, les surfaces actives maximales obtenues sont obtenues avec des particules greffées (cf. essai 6, à savoir l'essai où les particules sont greffées par un polymère comprenant une masse moléculaire de 359 000 g/mol et un taux massique de greffage de 3,88%) comparativement aux particules non greffées (cf. essai 12), avec un gain de surface active de 19%. Concernant l'activité électrochimique vis-à-vis de la réduction de l'oxygène (jₖ), celle-ci est de 8,63 mA/cm² pour les particules greffées de l'essai 6 contre 4,59 mA/cm² pour les particules non greffées de l'essai 12, ce qui représente une augmentation de 88%.

Ceci contribue à démontrer que le greffage de polymère non seulement ne réduit pas l'accessibilité de la surface de platine mais, au contraire, exacerbe cette dernière.

### EXEMPLE 9

Dans cet exemple, les particules obtenues aux essais 6, 7, 8 et 9 de l'exemple 4 ont été testées en pile dans les mêmes conditions d'assemblage et de test que celles explicitées à l'exemple 5 ci-dessus.

Les résultats sont reportés sur la figure 3 jointe en annexe, qui illustre, d'une part, les courbes de polarisation représentant l'évolution de la tension E (en V) en fonction de la densité de courant D (en A/cm²) (respectivement courbes a'), b'), c') et d') pour des piles comportant des particules des essais 6, 7, 8 et 9) et les courbes de puissance représentant l'évolution de densité de puissance P (en kW/cm²) en fonction de la densité de courant D (en A/cm²) (respectivement courbes a), b), c) et d) pour des piles comportant des particules des essais 6, 7, 8 et 9).

Il est observé des résultats particulièrement intéressants avec les particules obtenues par la méthode de synthèse polyol par voie micro-onde et, en particulier, celles greffées par des polymères de forte masse moléculaire et avec des taux de greffage relativement faibles.

La puissance maximale obtenue avec les particules les plus performantes est près de 400 % plus importante que dans le cas des particules obtenues par la synthèse eau-dans-huile. En effet, en se référant à la courbe a) (obtenue avec les particules de l'essai 6), la densité de puissance maximale obtenue est d'environ 1 kW/cm², alors que la densité de puissance maximale obtenue est d'environ 0,25 kW/cm² (obtenue avec les particules de l'essai 13, comme l'atteste la courbe b de la figure 1).

## Revendications

1. Procédé de préparation de particules de platine liées à un matériau carboné, lesdites particules étant greffées par des greffons consistant en au moins un polymère comprenant au moins un motif répétitif styrénique porteur d'au moins un groupe conducteur de protons, ledit procédé comprenant :
a) une étape de préparation desdites particules de platine liées à un matériau carboné comprenant une opération de chauffage sous micro-ondes d'un mélange comprenant un sel de platine, ledit matériau carboné et au moins un composé polyol, moyennent quoi sont obtenues lesdites particules ;
b) une étape de préparation d'au moins un polymère éthylénique par polymérisation ATRP d'un monomère éthylénique avec un amorceur ATRP répondant à la formule (I) suivante : 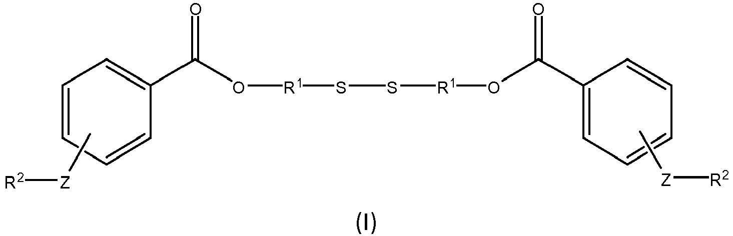 dans laquelle :
- les groupes R¹ représentent, indépendamment l'un de l'autre, un groupe espaceur organique ;
- les groupes Z représentent, indépendamment l'un de l'autre, une liaison simple ou un groupe espaceur organique ;
- les groupes R² représentent, indépendamment l'un de l'autre, un atome d'halogène ;
le polymère résultant répondant à la formule (II) suivante : 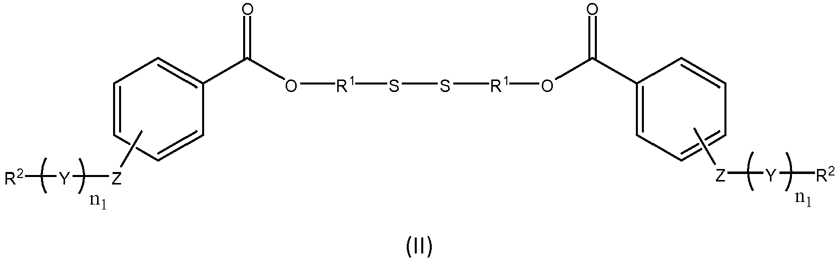 dans laquelle Y correspond au motif répétitif styrénique porteur d'au moins un groupe conducteur de protons et n₁ au nombre de répétition du motif répétitif pris entre parenthèses, les R¹, R² et Z étant tels que définis ci-dessus ;
c) une étape de mise en contact des particules obtenues en a) avec le polymère obtenu en b), moyennant quoi l'on obtient des particules greffées par des greffons de formule (III) suivante : 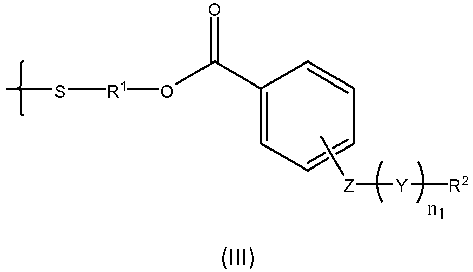 l'accolade indiquant l'endroit par lequel les greffons sont liés, de manière covalente, aux particules et les R¹, R², Z, Y et n₁ étant tels que définis ci-dessus.

2. Procédé selon la revendication 1, dans lequel le sel de platine est un sel d'halogénure de platine.

3. Procédé selon la revendication 1 ou 2, dans lequel le matériau carboné est du graphite, du noir de carbone, des fibres de carbone, des tubes de carbone ou du graphène.

4. Procédé selon l'une quelconque des revendications précédentes, dans lequel le matériau carboné est du noir de carbone.

5. Procédé selon l'une quelconque des revendications précédentes, dans lequel le composé polyol est un composé hydrocarboné comprenant au moins deux atomes de carbone porteurs chacun d'au moins un groupe -OH.

6. Procédé selon l'une quelconque des revendications précédentes, dans lequel l'étape a) est réalisée à un pH basique.

7. Procédé selon l'une quelconque des revendications précédentes, dans lequel R¹ et Z représentent, indépendamment l'un de l'autre, un groupe alkylène.

8. Procédé selon l'une quelconque des revendications précédentes, dans lequel les groupes -Z-R² sont situés en position *para* par rapport aux groupes -COO-.

9. Procédé selon l'une quelconque des revendications précédentes, dans lequel le groupe conducteur de protons est un groupe acide sulfonique -SO₃H, un groupe acide carboxylique -CO₂H ou un groupe acide phosphonique -PO₃H₂, ces groupes pouvant être présents éventuellement sous forme de sels.

10. Procédé selon l'une quelconque des revendications précédentes, dans lequel le monomère styrénique est un monomère de formule (V) suivante : 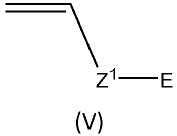 dans laquelle :
- Z¹ correspond à un groupe phénylène ; et
- E correspond à un groupe conducteur de protons, éventuellement sous forme d'un sel.

11. Procédé selon l'une quelconque des revendications précédentes, dans lequel le monomère styrénique est un monomère styrènesulfonate de sodium.

12. Particules de platine susceptibles d'être obtenues par un procédé selon l'une quelconque des revendications 1 à 11, lesdites particules de platine étant liées à un matériau carboné et étant greffées par des greffons de formule (III) suivante : 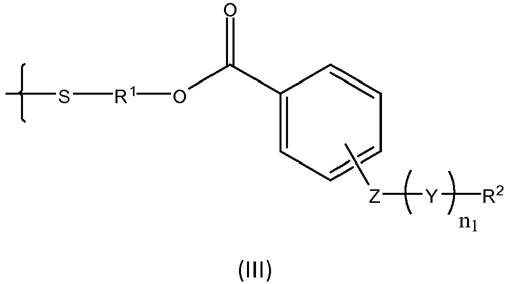 dans laquelle R¹, R², Z, Y et n₁ répondent aux mêmes définitions que celles données à la revendication 1.

13. Particules selon la revendication 12, dans lesquelles Y est un motif répétitif issu de la répétition d'un monomère styrènesulfonate de sodium.

14. Electrode comprenant des particules telles que définies selon l'une quelconque des revendications 12 ou 13.

15. Pile à combustible comprenant au moins un assemblage électrode-membrane-électrode, dans lequel au moins une de ses électrodes est une électrode telle que définie à la revendication 14.

## Patentansprüche

1. Verfahren zur Herstellung von Platinpartikeln, die an ein Kohlenstoffmaterial gebunden sind, wobei die Partikel mittels Pfröpflingen gepfropft sind, die aus wenigstens einem Polymer bestehen, umfassend wenigstens ein sich wiederholendes Styrolmotiv, das Träger wenigstens einer protonenleitenden Gruppe ist, wobei das Verfahren Folgendes umfasst:
a) einen Schritt der Herstellung der Platinpartikel, die an ein Kohlenstoffmaterial gebunden sind, umfassend eine Operation des Mikrowellenheizens einer Mischung, umfassend ein Platinsalz, das Kohlenstoffmaterial und wenigstens eine Polyolverbindung, wodurch die Partikel erhalten werden;
b) einen Schritt der Herstellung wenigstens eines Ethylenpolymers durch ATRP-Polymerisation eines Ethylenmonomers mit einem ATRP-Initiator, der der nachfolgenden Formel (I) genügt: 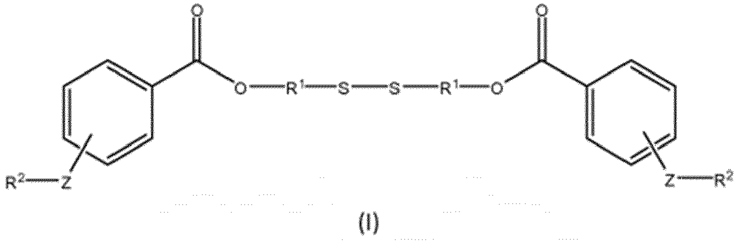 wobei:
- die Gruppen R¹ unabhängig voneinander eine organische Abstandhaltergruppe repräsentieren;
- die Gruppen Z unabhängig voneinander eine Einfachbindung oder eine organische Abstandhaltergruppe repräsentieren;
- die Gruppen R² unabhängig voneinander ein Halogenatom repräsentieren;
wobei das resultierende Polymer der nachfolgenden Formel (II) genügt: 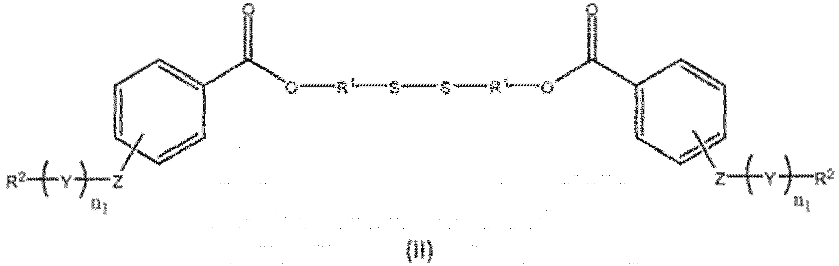 wobei Y dem sich wiederholenden Styrolmotiv entspricht, das Träger wenigstens einer protonenleitenden Gruppe ist, und n₁ der Wiederholungszahl des zwischen Klammern gesetzten sich wiederholenden Motivs, wobei die R¹, R² und Z wie oben definiert sind;
c) einen Schritt des Inkontaktbringens der in a) erhaltenen Partikel mit dem in b) erhaltenen Polymer, wodurch man Partikel erhält, die durch Pfröpflinge der nachfolgenden Formel (III) gepropft sind: 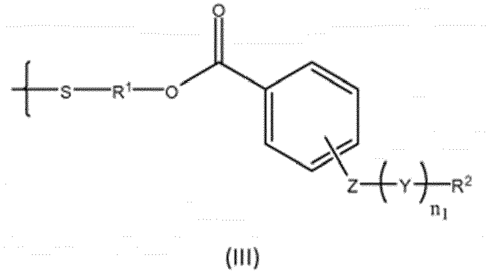 wobei die geschweifte Klammer den Ort angibt, an dem die Pfröpflinge in kovalenter Weise an die Partikel gebunden sind, und wobei die R¹, R², Z, Y und n₁ wie oben definiert sind.

2. Verfahren nach Anspruch 1, bei dem das Platinsalz ein Platinhalogenidsalz ist.

3. Verfahren nach Anspruch 1 oder 2, bei dem das Kohlenstoffmaterial Graphit, Kohlenstoffschwarz, Kohlenstofffasern, Kohlenstoffröhrchen oder Graphen ist.

4. Verfahren nach einem der vorhergehenden Ansprüche, bei dem das Kohlenstoffmaterial Kohlenstoffschwarz ist.

5. Verfahren nach einem der vorhergehenden Ansprüche, bei dem die Polyolverbindung eine Kohlenwasserstoffverbindung ist, die wenigstens zwei Kohlenstoffatome umfasst, die jeweils Träger wenigstens einer Gruppe-OH sind.

6. Verfahren nach einem der vorhergehenden Ansprüche, bei dem der Schritt a) bei einem basischen pH realisiert wird.

7. Verfahren nach einem der vorhergehenden Ansprüche, bei dem R¹ und Z unabhängig voneinander eine Alkylengruppe repräsentieren.

8. Verfahren nach einem der vorhergehenden Ansprüche, bei dem die Gruppen -Z-R² bezogen auf die Gruppen -COO- an einer Para-Position angeordnet sind.

9. Verfahren nach einem der vorhergehenden Ansprüche, bei dem die protonenleitende Gruppe eine Sulfonsäuregruppe -SO₃H ist, eine Carbonsäuregruppe -CO₂H, oder eine Phosphonsäuregruppe -PO₃H₂, wobei diese Gruppen gegebenenfalls in Form von Salzen vorhanden sein können.

10. Verfahren nach einem der vorhergehenden Ansprüche, bei dem das Styrolmonomer ein Monomer der nachfolgenden Formel (V) ist: 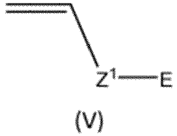 wobei:
- Z¹ einer Phenylengruppe entspricht; und
- E einer protonenleitenden Gruppe entspricht, gegebenenfalls in Form eines Salzes.

11. Verfahren nach einem der vorhergehenden Ansprüche, bei dem das Styrolmonomer ein Natriumstyrolsulfonatmonomer ist.

12. Platinpartikel, die durch ein Verfahren nach einem der Ansprüche 1 bis 11 erhältlich sind, wobei die Platinpartikel an ein Kohlenstoffmaterial gebunden und durch Pfröpflinge der nachfolgenden Formel (III) gepfropft sind: 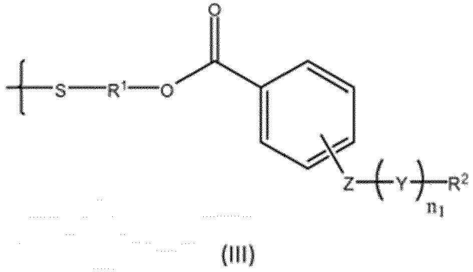 wobei R¹, R², Z, Y und n₁ den gleichen Definitionen wie den bei Anspruch 1 gegebenen genügen.

13. Partikel nach Anspruch 12, bei denen Y ein sich wiederholendes Motiv ist, das aus der Wiederholung eines Natriumstyrolsulfonatmonomers stammt.

14. Elektrode, umfassend Partikel wie in einem der Ansprüche 12 oder 13 definiert.

15. Brennstoffzelle, umfassend wenigstens eine Anordnung Elektrode-Membran-Elektrode, wobei wenigstens eine ihrer Elektroden eine Elektrode wie in Anspruch 14 definiert ist.

## Claims

1. Method for preparing platinum particles bonded to a carbon material, said particles being grafted with grafts consisting of at least one polymer comprising at least one styrene repeating unit having at least one proton-conducting group, said method comprising:
a) a step of preparing said platinum particles bonded to a carbon material comprising an operation of heating under microwaves of a mixture comprising a platinum salt, said carbon material and at least one polyol compound, subject to which said particles are obtained;
b) a step of preparing at least one ethylene polymer by ATRP polymerisation of an ethylene monomer with an ATRP initiator having the following formula (I): 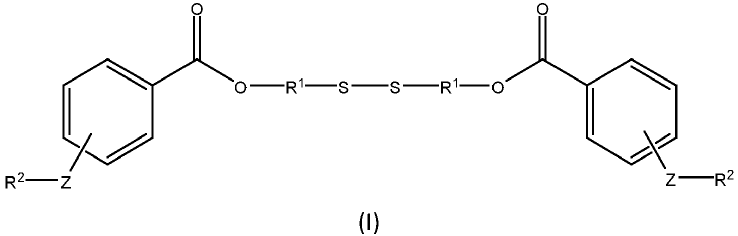 wherein:
- the R¹ groups represent, independently of one another, an organic spacer group;
- the Z groups represent, independently of one another, a single bond or an organic spacer group;
- the R² groups represent, independently of one another, a halogen atom;
the resulting polymer having the following formula (II): 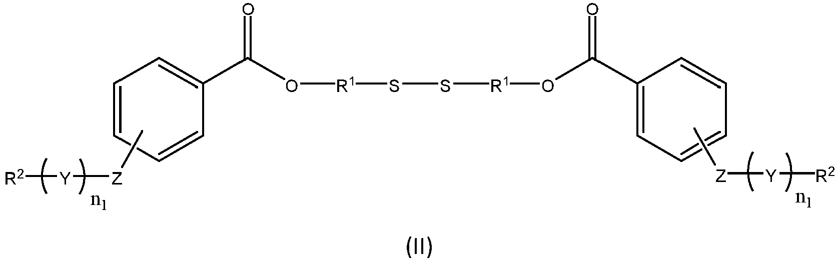 wherein Y corresponds to the styrene repeating unit having at least one proton-conducting group and n₁ to the repetition number of the repeating unit taken in parentheses, the R¹, R² and Z being such as defined hereinabove;
c) a step of contacting particles obtained in a) with the polymer obtained in b), subject to which particles are obtained grafted with grafts having the following formula (III): 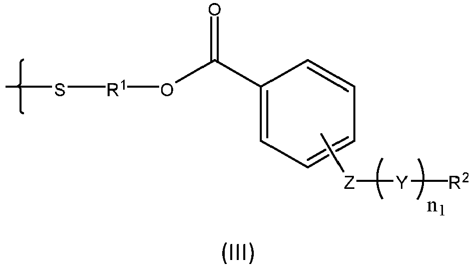 the brace indicating the location where the grafts are bonded, covalently, to the particles and the R¹, R², Z, Y and n₁ being such as defined hereinabove.

2. Method according to claim 1, wherein the platinum salt is a platinum halide salt.

3. Method according to claim 1 or 2, wherein the carbon material is graphite, carbon black, carbon fibres, carbon tubes or graphene.

4. Method as claimed in any preceding claim, wherein the carbon material is carbon black.

5. Method as claimed in any preceding claim, wherein the polyol compound is a hydrocarbon compound comprising at least two carbon atoms each having at least one -OH group.

6. Method as claimed in any preceding claim, wherein the step a) is carried out at a basic pH.

7. Method as claimed in any preceding claim, wherein R¹ and Z represent, independently of one another, an alkylene group.

8. Method as claimed in any preceding claim, wherein the -Z-R² groups are located in *para* position with respect to the -COO- groups.

9. Method as claimed in any preceding claim, wherein the proton-conducting group is a sulphonic acid group -SO₃H, a carboxylic acid group -CO₂H or a phosphonic acid group -PO₃H₂, these groups being able to be present optionally in the form of salts.

10. Method as claimed in any preceding claim, wherein the styrene monomer is a monomer having the following formula (V): 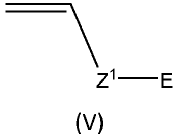 wherein:
- Z¹ corresponds to a phenylene group; and
- E corresponds to a proton-conducting group, optionally in the form of a salt.

11. Method as claimed in any preceding claim, wherein the styrene monomer is a sodium styrene sulphonate monomer.

12. Platinum particles being able to be obtained by a method according to any of claims 1 to 11, said platinum particles being bonded to a carbon material and being grafted with grafts having the following formula (III): 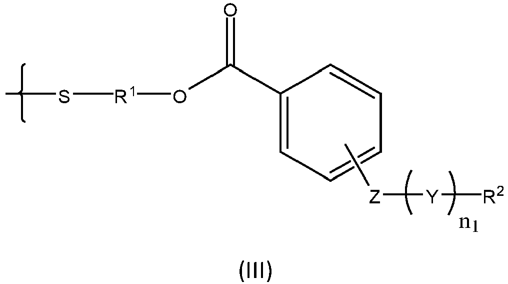 wherein R¹, R², Z, Y and n₁ have the same meaning as those given in claim 1.

13. Particles according to claim 12, wherein Y is a repeating unit coming from the repetition of a sodium styrene sulphonate monomer.

14. Electrode comprising particles such as defined according to any of claims 12 or 13.

15. Fuel cell comprising at least one electrode-membrane-electrode assembly, wherein at least one of its electrodes is an electrode such as defined in claim 14.
